# EUROPEAN PATENT SPECIFICATION

(11) **EP 2 223 684 B1**
(45) Date of publication and mention of the grant of the patent: **27.03.2013**
(21) Application number: 08855638.6
(22) Date of filing: 21.11.2008
(51) Int. Cl.: A61K 9/16, A61K 9/50, A61K 9/00, A61K 38/31

(54) **PHARMACEUTICAL COMPOSITION WITH PROLONGED RELEASE OF OCTREOTIDE ACETATE**
PHARMAZEUTISCHE ZUSAMMENSETZUNG MIT VERLÄNGERTER FREISETZUNG VON OCTREOTID ACETAT
COMPOSITION PHARMACEUTIQUE À LIBÉRATION PROLONGÉE DE OCTRÉOTIDE ACÉTATE

(30) Priority: 23.11.2007 ES 200703103
(43) Date of publication of application: 01.09.2010
(73) Proprietor: GP Pharm S.A., 08777 Sant Quinti Mediona Barcelona (ES)
(72) Inventor: PARENTE DUEÑA, Antonio, E-08960 Sant Just Desvern (Barcelona) (ES); SAVULSKY, Claudio, E-08960 San Just Desvern (Barcelona) (ES); GARCES GARCES, Josep, E-08760 Martorell (Barcelona) (ES); MIS VIZCAINO, Ricard, E-08010 Barcelona (ES)
(74) Representative: Carvajal y Urquijo, Isabel
(86) International application number: PCT/ES2008/000731
(87) International publication number: WO 2009/068708

(56) References cited:
- EP-A- 1 151 746
- WO-A-97/26015
- WO-A-2004/112752
- BLANCO-PRETO M J ET AL: "In vitro and in vivo evaluation of a somatostatin analogue released from PLGA microspheres" JOURNAL OF CONTROLLED RELEASE, ELSEVIER, AMSTERDAM, NL, vol. 67, no. 1, 1 June 2000 (2000-06-01), pages 19-28, XP004196069 ISSN: 0168-3659
- YAVUZ MELEK N ET AL: "The efficacy of octreotide in the therapy of acute radiation-induced diarrhea: a randomized controlled study." INTERNATIONAL JOURNAL OF RADIATION ONCOLOGY, BIOLOGY, PHYSICS 1 SEP 2002, vol. 54, no. 1, 1 September 2002 (2002-09-01), pages 195-202, XP002527245 ISSN: 0360-3016

## Description

### Field of the Invention

The present invention is generally comprised within the field of the biomedicine and it particularly relates to the use of a pharmaceutical composition of somatostatin, or an analog thereof, with a sustained release for approximately one week.

### Background of the Invention

It has been known for some time that diarrhea is one of the side effects associated to chemotherapy and to abdominal and/or pelvic radiotherapy in the treatment of cancer and that this side effect in many cases involves the hospitalization of the patient and the reduction of the treatment dose and even the interruption of treatment if it threatens the patient's life due to dehydration and the loss of electrolytes (Journal of Clinical Oncology, 2004, 22(14), 2918-26).

More than half the patients treated with 5-fluorouracil, cisplatin, irinotecan, capecitabine, leucovorin, tegafur, cyclophosphamide or methotrexate, among other chemotherapeutic agents, or subjected to abdominal and/or pelvic radiotherapy suffer from diarrhea (Annals of Oncology, 2001, 12(2), 227-9), and particularly from severe grade 3-4 diarrhea (according to the *National Cancer Institute Common Toxicity Criteria*) 8 hours after the administration of the chemotherapeutic agents or the application of radiotherapy.

Once the treatment of severe diarrhea associated to chemotherapy or to abdominal and/or pelvic radiotherapy begins, in most cases it does not last for more than one week.

It is known in the state of the art that the administration of derivatives of somatostatin prior, simultaneous to or after the administration of chemotherapeutic agents or pelvic radiotherapy serves to stop or reduce the intensity of diarrhea associated to these agents or to radiotherapy (International Journal of Radiation Oncology, Biology, Physics, 2002, 54(1), 195-202). Thus for example documents US 6395708 B1 and US 6159935 B1 relate to a method of preventing diarrhea caused by the administration of irinotecan comprising the administration of octreotide. According to the description, octreotide acetate is administered in the form of a slow release composition of octreotide Sandostatin LAR^{®} (1 month release depot).

Particularly, document US 6214792 B1 describes a method of treating acute and severe diarrhea comprising the administration of octreotide in a sufficient amount to alleviate diarrhea without causing bradycardia. According to the description, octreotide is parenterally administered every 12 hours together with an anticholinergic agent.

Document WO 02/074042 A2 describes a preparation comprising an epothilone for treating solid tumors and an antidiarrheal agent for treating diarrhea associated to the administration of epothilones. Octreotide is mentioned among the antidiarrheal agents and it is administered either as an immediate release composition or as the slow release composition of octreotide Sandostatin LAR^{®} (1 month release depot).

Furthermore, documents WO 2005/117830 A1 and WO 2006/075124 A1 describe a slow release, non-polymeric preformulation comprising octreotide, diacylglycerol, phosphatidylcholine and an organic solvent. This formulation can be used for treating diarrhea induced by chemotherapy and is administered every 20-90 days. However, the preformulation with octreotide can exceptionally be administered every 2-8 weeks in the treatment of advanced tumors.

There are also other documents which mention the administration of octreotide along with other active ingredients for treating diarrhea. Thus for example document WO 2005/105080 A1 relates to a method of treating diarrhea in a patient with cancer comprising the administration of an amide of 3-[4-bromo-2,6-difluorobenzyloxy)-5-(3-(4-pyrrolidin-1-yl-butyl)-ureido]-isothiazol-4-carboxylic acid together with octreotide, among other antidiarrheal agents. According to this document, octreotide can be administered by means of an oral sustained release composition, oral capsules, powder and oral solutions, or by means of parenteral injection as a solution, suspension or emulsion.

In addition, document EP 1040829 A2 describes a pharmaceutical composition comprising octreotide and a phosphodiesterase PDE-4 inhibitor for treating or preventing stasis resulting from hypomotility in the stomach caused by a therapeutic agent causing gastric hypomotility or gastrointestinal disorders.

Document WO 99/11277 A1 describes a method of alleviating a gastrointestinal disorder, including diarrhea and acute diarrhea, and comprising the suppression of gastric secretion and additionally treatment with an antidiarrheal agent such as octreotide, in which the latter is administered in an immediate release formulation. Likewise, document WO 2004/006902 A1 describes a composition for preventing or treating a gastrointestinal disorder comprising an amino-ether and/or amino-ester and a gastric secretion inhibitor, among which octreotide is mentioned and is administered either as an immediate release composition or as the slow release composition of octreotide Sandostatin LAR^{®} (1 month release depot).

Finally, the use of octreotide to alleviate and/or eliminate diarrhea associated to other pathologies and/or disorders is known in the state of the art. For example, documents US 5789411 A, US 5919760 A and WO 99/63935 A2 relate to the administration of octreotide to suppress or alleviate diarrhea associated to a drug detoxification method, in which octreotide is administered every 12 hours.

The use of octreotide to alleviate and/or eliminate grade 3-4 diarrhea as one of the symptoms of AIDS (AIDS, 1991, 5(6), 765-7; Scandinavian Journal of Infectious Diseases, 1994, 26(1), 55-7), grade 3-4 diarrhea associated to acute gastrointestinal graft-versus-host disease (Journal of Pediatric Hematology / Oncology, 2000, 22(4), 344-50), or diarrhea associated to other gastrointestinal disorders such as ulcerative colitis, collagenous colitis, microscopic colitis, lymphocytic colitis, Crohn's disease or diarrheas of an infectious viral or bacterial origin, is additionally known.

The aforementioned immediate release and slow release compositions of octreotide are commercial compositions. Among the immediate release compositions the Sandostatin^{®} brand stands out, which is obtained as described in document GB 2208200 A, i.e. an immediate release pharmaceutical composition comprising a lactic acid buffer and an analog of somatostatin such as octreotide. The composition marketed under the Sandostatin LAR^{®} brand stands out among the slow release compositions, and it is administered as described in document GB 2265311 A, i.e., a slow release composition of octreotide in a biodegradable and biocompatible polymeric support. As asserted in this document, biodegradation of the polymer ranges from several weeks to one or two months, which make a depot formulation made therefrom suitable for a one month's release. In all the examples of said document it is observed that plasma octreotide levels of 0.5 ng/ml were measured at least during 21 days, even reaching up to 42 days according to other examples.

Furthermore, document WO 2007/071395 A1 describes sustained release compositions of octreotide with two or more polylactic-co-glycolic polymers (PLGA), in which the release of active ingredient extends over a period of more than 3 months, preferably between 3 and 6 months.

As previously mentioned, severe grade 3-4 diarrhea associated to chemotherapy lasts for approximately one week in most cases. However, the need for pharmaceutical compositions of octreotide with a release only for approximately one week had not been considered. For this reason the pharmaceutical compositions of octreotide known in the state of the art and used for this treatment are either immediate release compositions and are administered continuously by intravenous route or are administered 2 or 3 times a day by subcutaneous route, or they are slow release compositions and are administered in a single intramuscular application for at least one month. Immediate release compositions have the drawback of the necessary patient hospitalization in the case of continuous intravenous administration, or the disadvantage of the high frequency of administration in the case of administration by subcutaneous route.

In addition, the total amount of octreotide that is released with the slow release formulation during one month is much higher than the amount of octreotide necessary for treating severe diarrhea during one week. In the slow release formulation the release of octreotide extends over one month, going well beyond the necessary treatment of approximately one week, and when it is recommended that the treatment with octreotide should stop in the 24 hours after the diarrhea disappears or if after 7 days no response to treatment with octreotide has been observed or if the patient develops an ileus or grade 4 toxicity due to the administration of octreotide *(*Journal of Clinical Oncology, 1997, 15 (11), 3350-4).

Document WO 2004/112752 discloses sustained release microspheres comprising octreotide and their production using a continuous one-step process.

There is therefore still a need to find an octreotide formulation for treating severe diarrhea associated to the treatment of cancer with chemotherapeutic agents and/or abdominal and/or pelvic radiotherapy which releases octreotide in a sufficient amount during all the days of treatment, in which the release of octreotide occurs only while the diarrhea lasts (approximately one week), with a lower frequency of administration and which prevents the reduction of the treatment dose or the interruption of treatment with chemotherapeutic agents and/or abdominal and/or pelvic radiotherapy. Moreover, the cases of patient hospitalization and the administration of an unnecessary amount of octreotide, or which may be ineffective, for treating diarrhea and which could further cause an ileus or grade 4 toxicity in the patient would also be prevented or reduced.

Compositions with the features of the composition object of the invention are also unknown in the state of the art for somatostatin or for other analogs of somatostatin such as vapreotide, lanreotide, seglitide, cortistatin or pasireotide.

Therefore, a stable pharmaceutical composition which releases a sufficient amount of somatostatin, or an analog of somatostatin, every day and only for approximately one week for treating diarrhea is an object of the present invention.

In addition to the aforementioned documents which describe compositions containing octreotide, document EP 1151746 A1 describes a pharmaceutical preparation of microcapsules of lactic-co-glycolic acid copolymer (PLGA) encapsulating somatostatin, or an analog of somatostatin, and incorporating citric acid esters for the purpose of modulating the release of the encapsulated drug without having to modify the composition of the polymer and preventing the high initial release of the drug.

The applicant of the present invention has surprisingly found that a pharmaceutical composition of microcapsules of lactic-co-glycolic acid copolymer (PLGA) or microcapsules of lactic-co-glycolic and polyethylene glycol copolymers (PLGA-PEG) incorporating citric acid esters and encapsulating somatostatin, or an analog of somatostatin, release a sufficient amount of somatostatin, or an analog of somatostatin, for approximately one week.

The present invention therefore provides a solution to the existing needs and comprises the use of a composition for administration in a single dose which releases octreotide in a sufficient amount for approximately one week, capable of treating and/or preventing diarrhea, and particularly preventing the reduction of the treatment dose or the interruption of the treatment with chemotherapeutic agents and/or abdominal and/or pelvic radiotherapy.

### Description of the Invention

The present invention relates to a pharmaceutical composition of octreotide acetate for administration in a single dose, for use in treating and/or preventing diarrhea, and to the use of said pharmaceutical composition for preparing a medicinal product for treating and/or preventing diarrhea.

Therefore a first aspect of the present invention relates to a microcapsule composition according to claim 1.

In a particular embodiment treating and/or preventing diarrhea relates to treating and/or preventing diarrhea associated to chemotherapy or associated to abdominal and/or pelvic radiotherapy in the treatment of cancer, diarrhea as one of the symptoms of AIDS, diarrhea associated to acute gastrointestinal graft-versus-host disease, diarrhea associated to ulcerative colitis, collagenous colitis, microscopic colitis, lymphocytic colitis, Crohn's disease and diarrheas of an infectious viral or bacterial origin, preferably grade 3-4 diarrhea associated to chemotherapy or associated to abdominal and/or pelvic radiotherapy in the treatment of cancer, grade 3-4 diarrhea as one of the symptoms of AIDS, grade 3-4 diarrhea associated to acute gastrointestinal graft-versus-host disease.

In the present invention, "cancer" relates to colorectal cancer, pancreatic cancer, esophageal cancer, stomach cancer, squamous tissue cancer, bladder cancer, prostate cancer, testicular cancer, ovarian cancer, cervical cancer, kidney cancer, breast cancer, lung cancer, mesothelial cancer, throat cancer, Kaposi's sarcoma, bone sarcoma, soft tissue sarcoma, Ewing's sarcoma, leukemia, neuroblastoma, lymphomas and brain tumors.

In the present invention, "treatment with chemotherapeutic agents" or "chemotherapy" relates to the treatment of cancer with at least one of the agents selected from the group consisting of 5-fluorouracil, cisplatin, oxaliplatin, carboplatin, irinotecan, capecitabine, leucovorin, tegafur, cyclophosphamide, methotrexate, epothilones, topotecan among others, or the therapeutic combinations of these drugs.

The dose of octreocide acetate that must be administered depends on several factors including, the condition and weight of the patient, the severity of the diarrhea and the exact duration of the release of octreotide acetate. The equivalent dose to octreotide that is administered for treating and/or preventing diarrhea ranges between 0.10 mg and 12 mg of octreotide, preferably between 0.20 mg and 6 mg of octreotide, and more preferably between 0.30 mg and 3 mg of octreotide.

In another particular embodiment, the sustained release of octreotide acetate preferably lasts a period of between 5 and 8 days, and more preferably 8 days from its administration.

In another particular embodiment, the microcapsule composition of PLGA comprising octreotide acetate includes the pharmaceutically acceptable auxiliary agents and/or supports necessary for the administration of the composition in the desired form. Included among the auxiliary agents and/or supports are excipients, thickening agents, diluents, solvents, dispersing agents, lyophilization improvement agents or adjuvants suitable for each route of administration and which are known by a person skilled in the art. The thickening agents include, but are not limited to, water-soluble polymers in such as those selected from the group consisting of modified celluloses, methylcellulose, ethylcellulose, hydroxyethylcellulose, hydroxyethylmethylcellulose, hydroxypropylcellulose, hydroxypropylmethylcellulose and carboxymethylcellulose, dextranes, gelatins, collagen, hyaluronic acid, polyethylene glycol or polyvinylpyrrolidone. The diluents and solvents include, but are not limited to, those selected from the group consisting of ethanol, polyethylene glycol, glycofurol, N-methyl-2-pyrrolidone, glycerol, propanediol, polypropylene glycol, benzyl alcohol or dimethylsulfoxide. The dispersing agents include, but are not limited to, the surfactants selected from the group consisting of polyoxyethylene sorbitan fatty acid monoesters (Tween^{®}, Emalex, Nikkol^{®}, Hodag, Dacol or Liposorb^{®}), sorbitan fatty acid monoesters (Span^{®}), polyethylene glycol 15-hydroxystearate (Solutol^{®} HS15), polyethylene glycol esters of fatty acids (Crodet, Cithrol, Kessco^{®}, Nikkol^{®}, Mapeg^{®}, Myrj, Tagat^{®}, Aldo^{®}, Capmul^{®}, Glycerox, Lactomul^{®} or Emerest^{®}), polyoxyethylene glycol esters (Emulphor^{®}), polyethoxylated castor oils (Cremophor^{®}, Emalex, Eumulgin^{®}, Nikkol^{®} or Simusol^{®}), polyglycerol esters of fatty acids (Nikkol Decaglyn, Polymuls, Caprol^{®}), polyethylene glycol ethers (Volpo or Brij^{®}), poloxamers (Lutrol^{®} or Pluronic^{®}), polyoxyethylene phenylethers (Triton^{®} or Igepal^{®}), or mixtures thereof. The lyophilization improvement agents include, but are not limited to, sugars such as those selected from the group consisting of mannitol, saccharose, glucose, fructose, lactose, trehalose, sucrose, dextrose, sorbitol, or glycine, gelatins, polyvinylpyrrolidone or mixtures thereof. Preferably, the composition for treating and/or preventing diarrhea additionally comprises one or several pharmaceutically acceptable excipients such as wetting agents, pH buffers, preservatives, bactericidal and fungicidal agents, retardants, absorption accelerators or any other excipient known by a person skilled in the art.

In another particular embodiment, the composition for treating and/or preventing diarrhea additionally comprises at least one agent selected from the group consisting of anticholinergic agents for treating and/or preventing bradycardia associated to the administration of octreotide, antidiarrheal agents for treating and/or preventing diarrhea and antiemetic agents for treating and/or preventing other symptoms such as nausea and/or vomiting. The anticholinergic agents for treating and/or preventing bradycardia associated to the administration of octreotide include, but are not limited to, those selected from the groups consisting of glycopyrrolate, atropine, benztropine, scopolamine, promethazine, diphenhydramine, dicyclomine, flavoxate, ipratropium, oxybutynin, pirenzepine, tiotropium, trihexyphenidyl, tolterodine, tropicamide, solifenacin, darifenacin, trimethaphan, atracurium, doxacurium, mivacurium, pancuronium, tubocurarine, vecuronium or suxamethonium. The antiemetic agents for treating and/or preventing other symptoms such as nausea and/or vomiting include, but are not limited to, those selected from the groups formed by steroids such as dexamethasone, dopamine antagonists such as domperidone, prochlorperazine, haloperidol, droperidol or metoclopramide; serotonin antagonists such as ondansetron, granisetron, alosetron, dolasetron, tropisetron or palonosetron or antihistamines such as meclizine or dimenhydrinate. The antidiarrheal agents for treating and/or preventing diarrhea include, but are not limited to, those selected from the group consisting of loperamide, diphenoxylate, difenoxin, laudanum, codeine, morphine, paregoric, hyoscyamine, dicyclomine, furazolidone, albumin tannate, lidamidine, mebiquine, trimebutine, fedotozine, bismuth subsalicylate, kaolin, pectin or attapulgite.

In another particular embodiment, the microcapsule composition of PLGA comprising octreotide acetate is presented in the the form of a unitary dose and is administered in the form of sterile solution or suspension by subcutaneous, intramuscular or intravenous parenteral route. In a preferred embodiment, the unitary dose composition is presented in the form of lyophilized powder which is reconstituted before its administration in the form of sterile suspension. In another preferred embodiment, the unitary dose composition is presented in the form of sterile solution or suspension which is injected as a gel forming *in situ* a biodegradable and biocompatible solid implant.

The preparation of the composition of the present invention for treating and/or preventing diarrhea can be carried out by any of the methods known in the state of the art. Particularly, the microcapsules of PLGA are prepared following any of the methods described in the literature, such as for example the methods described in documents US 3773919 A1, US 4728721 A1 or US 4849229 A1.

In a second aspect, this invention relates to the use of a microcapsule composition of PLGA comprising octreotide acetate, in preparing a medicinal product for treating and/or preventing diarrhea, characterized in that the microcapsules of PLGA sustainedly release a therapeutically effective amount of octreotide acetate, for a period of between 3 and 10 days from the administration of the medicinal product.

In a particular embodiment, treating and/or preventing diarrhea relates to treating and/or preventing diarrhea associated to chemotherapy or associated to abdominal and/or pelvic radiotherapy in the treatment of cancer, diarrhea as one of the symptoms of AIDS, diarrhea associated to acute gastrointestinal graft-versus-host disease, diarrhea associated to ulcerative colitis, collagenous colitis, microscopic colitis, lymphocytic colitis, Crohn's disease and diarrheas of an infectious viral or bacterial origin, preferably grade 3-4 diarrhea associated to chemotherapy or associated to abdominal and/or pelvic radiotherapy in the treatment of cancer, grade 3-4 diarrhea as one of the symptoms of AIDS, grade 3-4 diarrhea associated to acute gastrointestinal graft-versus-host disease, and more preferably grade 3-4 diarrhea associated to chemotherapy or associated to abdominal and/or pelvic radiotherapy in the treatment of cancer.

The dose of octreotide acetate which must be administered depends on several factors including the condition and weight of the patient the severity of the diarrhea and the exact duration of the release of octreotide acetate. The equivalent dose to octreotide which is administered for treating and/or preventing diarrhea ranges between 0.10 mg and 12 mg of octreotide, preferably between 0.20 mg and 6 mg of octreotide and more preferably between 0.30 mg and 3 mg of octreotide.

In another particular embodiment, the sustained release of octreotide acetate preferably lasts for a period of between 5 and 8 days, and more preferably 8 days from its administration.

In another particular embodiment, the microcapsule composition of PLGA comprising octreotide acetate includes the pharmaceutically acceptable auxiliary agents and/or supports necessary for the administration of the composition in the desired form. The auxiliary agents and/or supports include excipients, thickening agents, diluents, solvents, dispersing agents, lyophilization improvement agents or adjuvants suitable for each route of administration and which are known for a person skilled in the art. The thickening agents include" but are not limited to" water-soluble polymers such as those selected from the group consisting of modified celluloses, methylcellulose, ethylcellulose, hydroxyethylcellulose, hydroxyethyl methylcellulose, hydroxypropylcellulose, hydroxypropyl methylcellulose and carboxymethylcellulose, dextrans, gelatins, collagen, hyaluronic acid, polyethylene glycol or polyvinylpyrrolidone. The diluents and solvents include" but are not limited to" those selected from the group consisting of ethanol, polyethylene glycol, glycofurol, N-methyl-2-pyrrolidone, glycerol, propanediol, polypropylene glycol, benzyl alcohol or dimethyl sulfoxide. The dispersing agents include" but are not limited to" the surfactants selected from the group consisting of polyoxyethylene sorbitan fatty acid monoesters (Tween^{®}, Emalex, Nikkol^{®}, Hodag, Dacol or Liposorb^{®}), sorbitan fatty acid monoesters (Span^{®}), polyethylene glycol 15-hydroxystearate (Solutol^{®} HS15), fatty acid polyethylene glycol esters (Crodet, Cithrol, Kessco^{®}, Nikkol^{®}, Mapeg^{®}, Myrj, Tagat^{®}, Aldo^{®}, Capmul^{®}, Glycerox, Lactomul^{®} or Emerest^{®}), polyoxyethylene glycol esters (Emulphor^{®}), polyethoxylated castor oils (Cremophor^{®}, Emalex, Eumulgin^{®}, Nikkol^{®} or Simusol^{®}), fatty acid polyglycerol esters (Nikkol Decaglyn, Polymuls, Caprol^{®}), polyethylene glycol ethers (Volpo or Brij^{®}), poloxamers (Lutrol^{®} or Pluronic^{®}), polyoxyethylene phenyl ethers (Triton^{®} or Igepal^{®}), or mixtures thereof. The lyophilization improvement agents include" but are not limited to" sugars such as those selected from the group consisting of mannitol, saccharose, glucose, fructose, lactose, trehalose, sucrose, dextrose, sorbitol, or glycine, gelatins, polyvinylpyrrolidone or mixtures thereof. Preferably, the composition for treating and/or preventing diarrhea additionally comprises one or several pharmaceutically acceptable excipients such as wetting agents, pH buffers, preservatives, bactericidal and fungicidal agents, retardants, absorption accelerators, or any other excipient known by a person skilled in the art.

In another particular embodiment, the composition for treating and/or preventing diarrhea additionally comprises at least one agent selected from the group consisting of anticholinergic agents for treating and/or preventing bradycardia associated to the administration of octreotide, antidiarrheal agents for treating and/or preventing diarrhea and antiemetic agents for treating and/or preventing other symptoms such as nausea and/or vomiting. The anticholinergic agents for treating and/or preventing the bradycardia associated to the administration of octreotide include" but are not limited to" those selected from the group consisting of glycopyrrolate, atropine, benztropine, scopolamine, promethazine, diphenhydramine, dicyclomine, flavoxate, ipratropium, oxybutynin, pirenzepine, tiotropium, trihexyphenidyl, tolterodine, tropicamide, solifenacin, darifenacin, trimethaphan, atracurium, doxacurium, mivacurium, pancuronium, tubocurarine, vecuronium or suxamethonium. The antiemetic agents for treating and/or preventing other symptoms such as nausea and/or vomiting include" but are not limited to" those selected from the groups consisting of steroids such as dexamethasone, dopamine antagonists such as domperidone, prochlorperazine, haloperidol, droperidol or metoclopramide; serotonin antagonists such as ondansetron, granisetron, alosetron, dolasetron, tropisetron or palonosetron or antihistamines such as meclizine or dimenhydrinate. The antidiarrheal agents for treating and/or preventing the diarrhea include" but are not limited to" those selected from the group consisting of loperamide, diphenoxylate, difenoxin, laudanum, codeine, morphine, paregoric, hyoscyamine, dicyclomine, furazolidone, albumin tannate, lidamidine, mebiquine, trimebutine, fedotozine, bismuth subsalicylate, kaolin, pectin or attapulgite.

In another particular embodiment, the microcapsule composition, of PLGA comprising octreotide acetate is presented in the form of unitary dose and is administered in the form of sterile solution or suspension by subcutaneous, intramuscular or intravenous parenteral route. In a preferred embodiment, the unitary dose composition is presented in the form of lyophilized powder which is reconstituted before its administration in the form of sterile suspension. In another preferred embodiment, the unitary dose composition is presented in the form of sterile solution or suspension which is injected as a gel forming *in situ* a biodegradable and biocompatible solid implant.

In another aspect, this invention relates to a method of treating and/or preventing diarrhea, which method comprises the administration of the medicinal product for preventing and/or treating diarrhea before, simultaneously with or after the administration of one or several chemotherapeutic agents and/or the application of abdominal and/or pelvic radiotherapy. In a preferred embodiment the administration of the medicinal product for preventing and/or treating diarrhea is carried out before or simultaneously with the administration of one or several chemotherapeutic agents and/or the application of abdominal and/or pelvic radiotherapy, and more preferably it is carried out before, for example 1 or 2 days before the administration of one or several chemotherapeutic agents and/or the application of abdominal and/or pelvic radiotherapy.

### Detailed Description of the Invention

A microcapsule composition of PLGA comprising octreotide acetate has surprisingly been found which is suitable for use in treating and/or preventing diarrhea, wherein the microcapsules of PLGA sustainedly release a therapeutically effective amount of octreotide acetate for a period of between 3 and 10 days from the administration of the medicinal product, and the use of said composition for preparing a medicinal product for treating and/or preventing diarrhea

### Examples

These examples intend to illustrate and never limit the invention

### Example 1

### Preparation of microcapsules of octreotide with an 8-day release profile

2 g of triethyl citrate and 1.5 g of lactic-co-glycolic polymer (mw=35000 with a lactic:glycolic ratio of 1:1) were dissolved in 50 ml of dichloromethane. Once the polymer had been dissolved, 20 mg of octreotide acetate were added and were suspended by sonication and then 70 g of silicone of 350 cts were added slowly and witch intense stirring.

The content of the reactor was then added in 2.5 l of n-heptane and stirred for 1 hour. Finally, the microcapsules were separated by filtration and dried under vacuum for 48 hours.

### Example 2 (comparative)

### Preparation of microcapsules of octreotide with a 6-day release profile

1.5 g of triethyl citrate, 1.2 g of lactic-co-glycolic polymer (mw=35000 with a lactic:glycolic ratio of 1:1) and 0.4 g of polyethylene glycol 200 were dissolved in 50 ml of dichloromethane. Once the polymers had been dissolved, 15 mg of octreotide acetate were added and were suspended by sonication and then 70 g of silicone of 350 cts were added slowly and with intense stirring.

The content of the reactor was then added in 2.5 l of n-heptane and stirred for 1 hour. Finally, the microcapsules were separated by filtration and dried under vacuum for 48 hours.

### Example 3

### In vitro test of the release of octreotide in the microcapsules of Example 1

10 mg of microcapsules containing octreotide obtained in Example 1 were weighed in several 10 ml-capacity plastic tubes provided with a stopper. 2 ml of 1/30 M phosphate buffer, pH=7.0, were added in each tube. Each tube was shaken to suspend the microcapsules in the buffer and they were placed in an oven at 37 °C.

The samples for the hydrolysis control were taken after 1 hour, 1 day, 2 days, 3 days, 6 days and 8 days. The analysis was carried out by means of HPLC by quantifying the peptide released in the supernatant or the residual peptide inside the microcapsules.

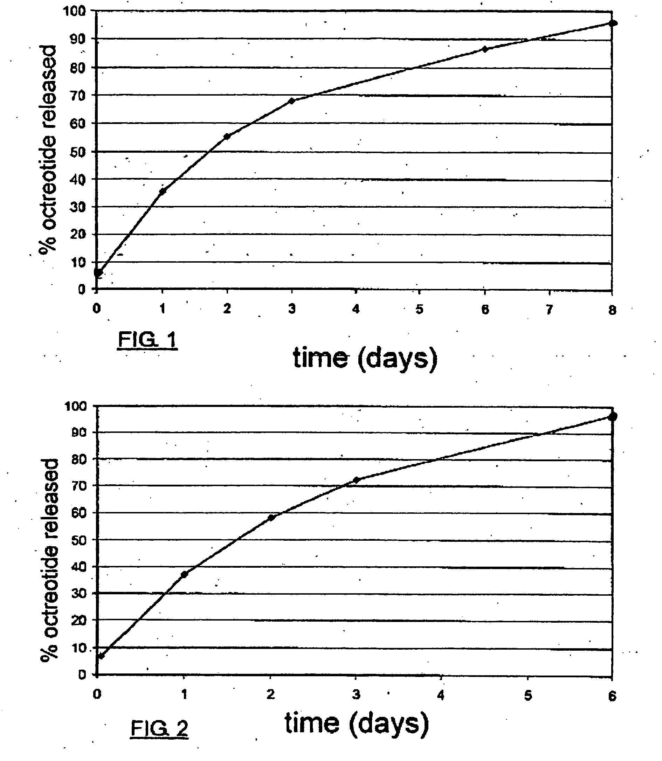

The result of this analysis is shown in Figure 1, which shows the hydrolysis of the microcapsules of Example 1 according to time.

### Example 4 (comparative)

### In vitro test of the release of octreotide in the microcapsules of Example 2

The test was carried out in the manner described in Example 3 but with the microcapsules of octreotide obtained in Example 2. The result of this analysis is shown in Figure 2, which shows the hydrolysis of the microcapsules of Example 2 according to time.

### Example 5

### Pharmaceutical composition of octreotide in microcapsules of PLGA.

The microcapsules were prepared in the manner described in Example 1. The thickening agents, lyophilization improvement agents and surfactants of Table 1 were added to these microcapsules, according to the usual methods known by persons skilled in the art, to form the composition of this example.

**Table 1**

| Ingredients | Amount per vial (mg) |
|---|---|
| Octreotide acetate | 2 |
| PLGA | 150 |
| Sodium carboxymethylcellulose | 30 |
| Triethyl citrate | 8 |
| Mannitol | 85 |
| Polysorbate Tween 80 | 2 |

This composition was stored in a vial for extemporaneous resuspension containing 2 ml of 0.8% mannitol in water.

### Example 6 (comparative)

### Pharmaceutical composition of octreotide in microcapsules of PLGA.

The microcapsules were prepared in the manner described in Example 1 but substituting triethyl citrate with acetyl triethyl citrate. The thickening agents, lyophilization improvement agents and surfactants of Table 2 were added to these microcapsules, according to the usual methods known by persons skilled in the art, to form the composition of this example.

**Table 2**

| Ingredients | Amount per vial (mg) |
|---|---|
| Octreotide acetate | 2 |
| PLGA | 150 |
| Sodium carboxymethylcellulose | 30 |
| Acetyl triethyl citrate | 10 |
| Lactose | 70 |
| Cremophor | 2 |

This composition was stored in a vial for extemporaneous resuspension containing 2 ml of 0.8% mannitol in water.

### Example 7: (comparative)

### Pharmaceutical composition of octreotide in microcapsules of PLGA.

The microcapsules were prepared in the manner described in Example 1 but substituting triethyl citrate with acetyl triethyl citrate. The solvents of Table 3 were added to these microcapsules, according to the usual methods known by the persons skilled in the art, to form the composition of this example.

**Table 3**

| Ingredients | Amount per vial (mg) |
|---|---|
| Octreotide acetate | 2 |
| PLGA | 150 |
| Glycofurol | 1 ml |
| Acetyl triethyl citrate | 10 |

This composition was stored in a syringe to be intramuscularly injected, forming a biodegradable and biocompatible solid implant.

### Example 8: (comparative)

### Pharmaceutical composition of octreotide in microcapsules of PLGA-PEG.

The microcapsules were prepared in the manner described in Example 2. The thickening agents, lyophilization improvement agents and surfactants of Table 4 were added to these microcapsules, according to the usual methods known by persons skilled in the art, to form the composition of this example.

**Table 4**

| Ingredients | Amount per vial (mg) |
|---|---|
| Octreotide acetate | 1.4 |
| PLGA-PEG | 160 |
| Sodium carboxymethylcellulose | 30 |
| Triethyl citrate | 9 |
| Mannitol | 85 |
| Polysorbate Tween 80 | 2 |

This composition was stored in a vial for extemporaneous resuspension containing 2 ml of 0.8% mannitol in water.

### Example 9: (comparative)

### Pharmaceutical composition of octreotide in microcapsules of PLGA-PEG.

The microcapsules were prepared in the manner described in Example 2 but substituting triethyl citrate with trioctyl citrate. The thickening agents, lyophilization improvement agents and surfactants of Table 5 were added to these microcapsules, according to the usual methods known by persons skilled in the art, to form the composition of this example.

**Table 5**

| Ingredients | Amount per vial (mg) |
|---|---|
| Octreotide acetate | 1.4 |
| PLGA-PEG | 160 |
| Hydroxypropylcellulose | 30 |
| Trioctyl citrate | 7 |
| Saccharose | 90 |
| Solutol HS15 | 2 |

This composition was stored in a vial for extemporaneous resuspension containing 2 ml of 0.8% mannitol in water.

### Example 10: (comparative)

### Pharmaceutical composition of octreotide in microcapsules of PLGA-PEG.

The microcapsules were prepared in the manner described in Example 2 substituting triethyl citrate with acetyl tributyl citrate. The solvents of Table 6 were added to these microcapsules, according to the usual methods known by persons skilled in the art, to form the composition of this example.

**Table 6**

| Ingredients | Amount per vial (mg) |
|---|---|
| Octreotide acetate | 1.4 |
| PLGA-PEG | 160 |
| Glycofurol | 1 ml |
| Acetyl tributyl citrate | 8 |

This composition was stored in a syringe to be intramuscularly injected, forming a biodegradable and biocompatible solid implant.

### Example 11: (comparative)

### Pharmaceutical composition of octreotide in microcapsules of PLGA-PEG.

The microcapsules were prepared in the manner described in Example 2 substituting triethyl citrate with acetyl tributyl citrate. The solvents of Table 7 were added to these microcapsules, according to the usual methods known by persons skilled in the art, to form the composition of this example.

**Table 7**

| Ingredients | Amount per vial (mg) |
|---|---|
| Octreotide acetate | 1.4 |
| PLGA-PEG | 160 |
| N-methyl-2-pyrrolidone | 1 ml |
| Acetyl tributyl citrate | 8 |

This composition was stored in a syringe to be intramuscularly injected, forming a biodegradable and biocompatible solid implant.

### Example 12: (comparative)

### Preparation of microcapsules of PLGA containing somatostatin with a 7-day release profile.

1.5 g of triethyl citrate and 1.5 g of lactic-co-glycolic polymer (mw=35000 with a lactic:glycolic ratio of 1:1) were dissolved in 50 ml of dichloromethane. Once the polymer had been dissolved, 0.4 g of somatostatin acetate were added arid were suspended by sonication and then 70 g of silicone of 350 cts were added slowly and with intense stirring.

The content of the reactor was then added in 2.5 l of n-heptane and stirred for 1 hour. Finally, the microcapsules were separated by filtration and dried under vacuum for 48 hours.

### Example 13: (comparative)

### Preparation of microcapsules of somatostatin with a polymer of PLGA-PEG.

1.6 g of tributyl citrate and 1.0 g of lactic-co-glycolic polymer (mw=35000 with a lactic:glycolic ratio of 1:1) and 0.6 g of polyethylene glycol 200 were dissolved in 50 ml of dichloromethane. Once the polymer had been dissolved, 0.4 g of somatostatin acetate were added and were suspended by sonication and then 70 g of silicone of 350 cts were added slowly and with intense stirring.

The content of the reactor was then added in 2.5 l of *n*-heptane and stirred for 1 hour. Finally, the microcapsules were separated by filtration and dried under vacuum for 48 hours.

### Example 14: (comparative)

### In vitro test of the release of somatostatin in the microcapsules of Example 12.

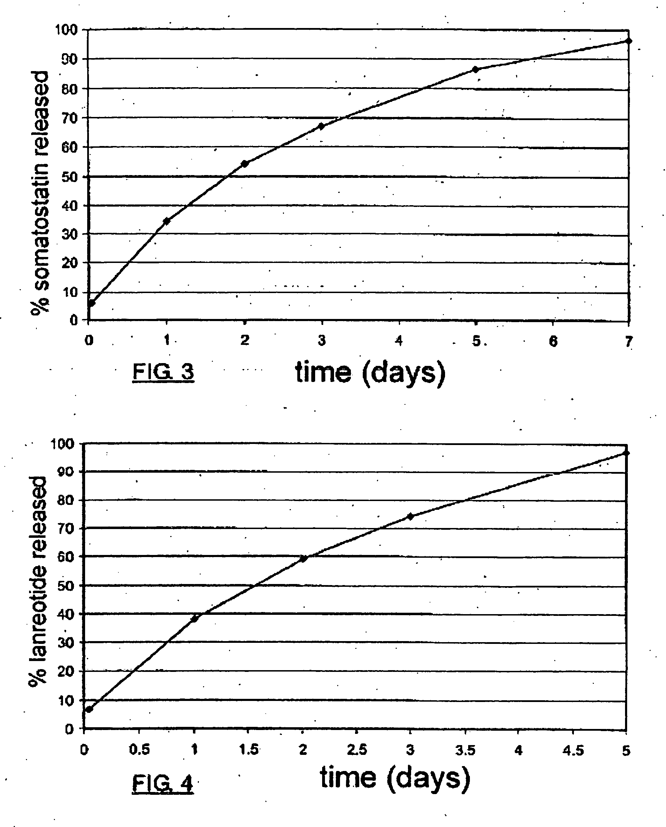

The test was carried out in the manner described in Example 3 but with the microcapsules of somatostatin obtained in Example 12. The result of this analysis is shown in Figure 3, which shows the hydrolysis of the microcapsules of Example 12 according to time.

### Example 15: (comparative)

### Pharmaceutical composition of somatostatin in microcapsules of PLGA.

The microcapsules were prepared in the manner described in Example 12. The thickening agents, lyophilization improvement agents and surfactants of Table 8 were added to these microcapsules, according to the usual methods known by persons skilled in the art, to form the composition of this example.

**Table 8**

| Ingredients | Amount per vial (mg) |
|---|---|
| Somatostatin acetate | 40 |
| PLGA | 150 |
| Hydroxyethyl methylcellulose | 30 |
| Triethyl citrate | 7 |
| Saccharose | 90 |
| Polysorbate Tween 80 | 2 |

This composition was stored in a vial for extemporaneous resuspension containing 2 ml of 0.8% mannitol in water.

### Example 16: (comparative)

### Pharmaceutical composition of somatostatin in microcapsules of PLGA-PEG.

The microcapsules were prepared in the manner described in Example 13. The solvents of Table 9 were added to these microcapsules, according to the usual methods known by persons skilled in the art, to form the composition of this example.

**Table 9**

| Ingredients | Amount per vial (mg) |
|---|---|
| Somatostatin acetate | 40 |
| PLGA-PEG | 160 |
| Glycofurol | 1 ml |
| Tributyl citrate | 8 |

This composition was stored in a syringe to be intramuscularly injected, forming a biodegradable and biocompatible solid implant.

### Example 17: (comparative)

### Preparation of microcapsules of PLGA containing lanreotide with a 7-day release profile.

1.5 g of triethyl citrate and 1.5 g of lactic-co-glycolic polymer (mw=50000 with a lactic:glycolic ratio of 1:1) were dissolved in 50 ml of dichloromethane. Once the polymer had been dissolved, 0.2 g of lanreotide acetate were added and were suspended by sonication and then 70 g of silicone of 350 cts were added slowly and with intense stirring.

The content of the reactor was then added in 2.5 l of n-heptane and stirred for 1 hour. Finally, the microcapsules were separated by filtration and dried under vacuum for 48 hours.

### Example 18: (comparative)

### Preparation of microcapsules of lanreotide with a polymer of PLGA-PEG.

1.5 g of triethyl citrate and 1.0 g of lactic-co-glycolic polymer (mw=50000 with a lactic:glycolic ratio of 1:1) and 0.6 g of polyethylene glycol 200 were dissolved in 50 ml of dichloromethane. Once the polymer had been dissolved, 0.2 g of lanreotide acetate were added and were suspended by sonication and then 70 g of silicone of 350 cts were added slowly and with intense stirring.

The content of the reactor was then added in 2.5 l of n-heptane and stirred for 1 hour. Finally, the microcapsules were separated by filtration and dried under vacuum for 48 hours.

### Example 19: (comparative)

### In vitro test of the release of lanreotide in the microcapsules of Example 17.

The test was carried out in the manner described in Example 3 but with the microcapsules of lanreotide obtained in Example 17. The result of this analysis is shown in Figure 4, which shows the hydrolysis of the microcapsules of Example 17 according to time.

### Example 20: (comparative)

### Pharmaceutical composition of lanreotide in microcapsules of PLGA.

The microcapsules were prepared in the manner described in Example 17. The thickening agents, lyophilization improvement agents and surfactants of Table 10 were added to these microcapsules, according to the usual methods known by persons skilled in the art, to form the composition of this example.

**Table 10**

| Ingredients | Amount per vial (mg) |
|---|---|
| Lanreotide acetate | 20 |
| PLGA | 150 |
| Hydroxypropyl methylcellulose | 30 |
| Triethyl citrate | 8 |
| Trehalose | 80 |
| Polysorbate Tween 80 | 2 |

This composition was stored in a vial for extemporaneous resuspension containing 2 ml of 0.8% mannitol in water.

### Example 21: (comparative)

### Pharmaceutical composition of lanreotide in microcapsules of PLGA-PEG.

The microcapsules were prepared in the manner described in Example 18. The solvents of Table 11 were added to these microcapsules, according to the usual methods known by persons skilled in the art, to form the composition of this example.

**Table 11**

| Ingredients | Amount per vial (mg) |
|---|---|
| Lanreotide acetate | 20 |
| PLGA-PEG | 160 |
| Polyethylene glycol 200 | 1 ml |
| Triethyl citrate | 8 |

This composition was stored in a syringe to be intramuscularly injected, forming a biodegradable and biocompatible solid implant.

## Claims

1. A microcapsule composition for sustained release of a therapeutically effective amount of octreotide acetate for a period between 3 to 10 days from the administration of said microcapsule composition, comprising lactic-co-glycolic acid copolymer (PLGA) of molecular weight 35000 Daltons, 5% triethyl citrate by weight with respect to the weight of the microcapsule, for use in treating and/or preventing diarrhea, wherein the lactic:glycolic ratio is 1:1.

2. The microcapsule composition according to claim 1, having a sustained release for a period between 5 to 8 days from the administration of said microcapsule composition.

3. The microcapsule composition according to claim 2, having a sustained release for a period of 8 days from the administration of said microcapsule composition.

4. The microcapsule composition according to any of previous claims, wherein the composition comprises at least one pharmaceutically acceptable auxiliary agent selected from the group consisting of thickening agents, lyophilization improvement agents and surfactants.

5. The microcapsule composition according to any of previous claims , wherein the diarrhea is selected from the group consisting of diarrhea associated to chemotherapy or associated to abdominal and /or pelvic radiotherapy in the treatment of cancer, diarrhea as one of the symptoms of AIDS, diarrhea associated to acute gastrointestinal graft- versus-host disease, diarrhea associated to ulcerative colitis, collagenous colitis, microscopic colitis, lymphocytic colitis, Crohn's disease, diarrheas of an infectious viral origin and diarrheas of a bacterial origin.

## Patentansprüche

1. Eine Mikrokapselzusammensetzung zur anhaltenden Freisetzung einer therapeutisch wirksamen Menge Ocreotidacetats über einen Zeitraum von 3 bis 10 Tagen seit der Verabreichung besagter Mikrokapselzusammensetzung, umfassend Milchsäure-co-Glykolsäure-Copolymer (PLGA) mit einem Molekulargewicht von 35000 Dalton, 5 Gew.-% Triethylcitrat hinsichtlich des Gewichts der Mikrokapsel, zur Verwendung in der Behandlung und/oder Vorbeugung von Diarrhöe, wobei das Verhältnis Milchsäure:Glykolsäure 1:1 ist.

2. Die Mikrokapselzusammensetzung nach Anspruch 1, wobei diese eine anhaltende Freisetzung über einen Zeitraum von 5 bis 8 Tagen seit der Verabreichung besagter Mikrokapselzusammensetzung aufweist.

3. Die Mikrokapselzusammensetzung nach Anspruch 2, wobei diese eine anhaltende Freisetzung über einen Zeitraum von 8 Tagen seit der Verabreichung besagter Mikrokapselzusammensetzung aufweist.

4. Die Mikrokapselzusammensetzung nach einem der vorhergehenden Ansprüche, wobei die Zusammensetzung zumindest einen pharmazeutischen verträglichen Hilfsstoff umfasst, ausgewählt aus der Gruppe bestehend aus Verdickungsmitteln, Mitteln zur Verbesserung der Lyophilisierung und Tenside.

5. Die Mikrokapselzusammensetzung nach einem der vorhergehenden Ansprüche, wobei die Diarrhöe aus der Gruppe ausgewählt ist, die aus mit Chemotherapie oder mit abdomineller und/oder pelvischer Strahlentherapie verbundene Diarrhöe bei der Krebsbehandlung, Diarrhöe als eines der Symptome von AIDS, mit akuter gastrointestinaler Transplantat-Wirt-Reaktion verbundene Diarrhöe, mit Colitis ulcerosa, kollagener Kolitis, mikroskopischer Kolitis, lymphozytärer Kolitis, Morbus Crohn verbundene Diarrhöe, Diarrhöen infektiösen viralen Ursprungs und Diarrhöen bakteriellen Ursprungs besteht.

## Revendications

1. Composition de microcapsules pour la libération prolongée d'une quantité thérapeutiquement effective d'acétate d'octreotide sur une période de 3 à 10 jours après l'administration de ladite composition de microcapsules, qui comprend un copolymère d'acide lactique-co-glycolique (PLGA) d'un poids moléculaire de 35000 Daltons, un 5 % en poids de citrate de triéthyle par rapport au poids de la microcapsule, pour son utilisation dans le traitement et/ou prévention de la diarrhée, dans laquelle le ratio d'acide lactique:glycolique est de 1:1.

2. Composition de microcapsules selon la revendication 1, ayant une libération prolongée sur une période de 5 à 8 jours après l'administration de ladite composition de microcapsules.

3. Composition de microcapsules selon la revendication 2, ayant une libération prolongée pendant sur une période de 8 jours après l'administration de ladite composition de microcapsules.

4. Composition de microcapsules selon l'une quelconque des revendications précédentes, dans laquelle la composition comprend au moins un agent auxiliaire pharmaceutiquement acceptable, sélectionné d'un groupe consistant en des agents épaississants, des agents d'amélioration de la lyophilisation et des agents tensioactifs.

5. Composition de microcapsules selon l'une quelconque des revendications précédentes, dans laquelle la diarrhée est sélectionné du groupe consistant en une diarrhée associée à une chimiothérapie ou associé à une radiothérapie abdominale et/ou pelvienne dans le traitement du cancer, une diarrhée en tant qu'un des symptômes du SIDA, une diarrhée associée à la maladie gastrointestinal aiguë du greffon contre l'hôte, une diarrhée associée à une colite ulcérative, une colite collagénose, une colite microscopique, une colite lymphocytaire, la maladie de Crohn, des diarrhées d'origine virale infectieuse et des diarrhées d'origine bactérienne.
